# EUROPEAN PATENT APPLICATION

(11) **EP 3 210 571 A1**
(43) Date of publication of application: **30.08.2017**
(21) Application number: 15851799.5
(22) Date of filing: 22.10.2015
(51) Int. Cl.: A61F 2/16

(54) **INTRAOCULAR LENS INJECTOR**

(30) Priority: 22.10.2014 JP 2014215337
(71) Applicant: Santen Pharmaceutical Co., Ltd., Higashiyodogawa-ku Osaka-shi Osaka 533-8651 (JP)
(72) Inventor: UENO, Katsuyuki, Osaka-shi Osaka 530-8552 (JP)
(74) Representative: Müller-Boré & Partner Patentanwälte PartG mbB
(86) International application number: PCT/JP2015/079793
(87) International publication number: WO 2016/063937

(57) **Abstract**

The purpose of the present invention is to provide an intraocular lens injector with which the tip side of a plunger can be finely formed while ensuring rigidity, and with which the occurrence of shaft deviation during contact with an intraocular lens can be effectively inhibited. A plunger (3) of this intraocular lens injector (1) is provided with: a tip-side shaft part (35) provided with an L-shaped section (36); a central shaft part (33) which is formed flat, and which is wider than the tip-side shaft part (35); and a part (40) to be restricted which is formed from the tip-side shaft part (35) to the central shaft part (33), and which protrudes from the flat surface at the central shaft part (33). Furthermore, an opening (70) in a main body (2) is provided with: a central opening (71) which is open in accordance with the shape of the central shaft part (33); and a restriction part (72) formed so as to protrude from the end surface of the central opening (71), in the thickness direction of the central shaft part (33), in accordance with the shape of the part (40) to be restricted. The part (40) to be restricted has a length in the axial direction which is set such that the part (40) to be restricted is restricted by the restriction part (72) in an interval from an initial position before the L-shaped section (36) begins to be in contact with a second lens support part (93) of an intraocular lens (90), to a position in which the L-shaped section (36) is in contact with the second lens support part (93).

## Description

### TECHNICAL FIELD

The present invention relates to an intraocular lens injector.

### BACKGROUND ART

Conventionally, in cataract surgery in which a clouded crystalline lens in the eye is replaced with an artificial intraocular lens, an intraocular lens injector has been used that delivers an intraocular lens set in a lens holder into the eye by pushing a plunger that was inserted into a main body. With the intraocular lens injector, since there is risk of insertion of the intraocular lens not being appropriately performed if contacting with the intraocular lens in a state in which the leading end of the plunger is deviating from the appropriate position, a shaft shake prevention structure may be provided for controlling the posture of the plunger. Patent Documents 1 to 3 are publications disclosing this type of intraocular lens injector. Patent Document 1 discloses a structure that prevents shaft shake in the left/right direction of the plunger by way of a shaft projecting part formed in a pair of rod shapes formed from a ceiling to the bottom of a lens holder. Patent Document 2 and Patent Document 3 disclose structures that prevent rotation of the plunger, by establishing the inner cylinder of a main body as a substantially semi-circular shape, and establishing the shape of the plunger also as a substantially semi-circular shape.
Patent Document 1: Japanese Unexamined Patent Application, Publication No. 2010-273985
Patent Document 2: Japanese Unexamined Patent Application, Publication No. 2010-82288
Patent Document 3: Japanese Unexamined Patent Application, Publication No. 2009-112355

### DISCLOSURE OF THE INVENTION

### Problems to be Solved by the Invention

The intraocular lens injector is configured so as to narrow as approaching towards a leading-end side for releasing into the eye by folding an optical part of the intraocular lens during the course of insertion. From the viewpoint of avoiding interference with the inner wall on the leading-end side of the intraocular lens injector, the leading-end side of the plunger is preferably formed to be thin. However, in the case of forming the leading-end side of the plunger to be thin, since an opening at the outlet of the main body must be formed according to the shape on the base-end side, which is thicker than the leading-end side, the gap between the leading-end side of the plunger and the opening becomes larger, and there is a risk of shaft shake occurring at the leading-end side of the plunger. In this point, although the configuration disclosed in Patent Document 1 can prevent shake in the left-right direction, it is not able to prevent shake in the vertical direction. In addition, although the configurations disclosed in Patent Document 2 and Patent Document 3 restrict the movement in the rotational direction of the plunger by way of the inner cylinder, in the case of the end face of the leading end of the plunger that contacts the intraocular lens being made smaller than the inside diameter of the inner cylinder, since shaft shake at the leading-end side will not be restricted, there has been a risk of not being able to prevent shaft shake at the moment when the intraocular lens contacts with the leading end of the plunger. Additionally, from the viewpoint of maintaining rigidity, if thickening the base-end side of the plunger and forming the leading-end side to be thin, the gap between the leading-end side of the plunger and the opening, which is formed in accordance with the shape of the base-end side, will become larger, and the problem of becoming difficult to prevent shaft shake at the leading-end side of the plunger also becomes an issue to be solved.

The present invention has an object of providing an intraocular lens injector that can form the leading-end side of the plunger to be thin while retaining rigidity, as well as can effectively prevent the occurrence of shaft shake during contact with the intraocular lens.

### Means for Solving the Problems

The present invention relates to an intraocular lens injector including: a main body formed in a cylindrical shape; a lens holder that is disposed at a leading-end side of the main body, and in which an intraocular lens is set; and a plunger that is inserted in the main body, and pushes out the intraocular lens set in the lens holder from an opening formed in the leading-end side of the main body to a leading-end side, in which the plunger includes: a leading-end part having a contact part that contacts with the intraocular lens; a flat part that is connected to a base-end side of the leading-end part, and is formed to be flat and wider than the leading-end part; and a restricted part that is formed along an axial direction of the plunger from the leading-end part to the flat part, and projects from a flat face at the flat part, in which the opening includes: a central opening that is opened in accordance with the shape of the flat part; and a restricting part that is formed so as to project in a thickness direction of the flat part from an end face of the central opening in accordance with the shape of the restricted part, and in which the restricted part has a length in the axial direction thereof set so as to be restricted by at least the restricting part from an initial position prior to the contact part starting contact with the intraocular lens until contacting.

It is preferable for the plunger to further include a second restricting part that projects along the axial direction of the plunger at a flat face on a side opposite to a face of the flat part on which the restricted part is formed; the opening to further include a second restricting part that is disposed on a side opposite to the restricting part to sandwich the central opening, and is formed in accordance with a shape of the second restricted part of the plunger; and the second restricted part to have a length in the axial direction thereof set so as to be restricted by at least the second restricting part from an initial position prior to the contact part starting contact with the intraocular lens until contacting.

It is preferable for the second restricted part to be formed continuously from the flat part until an end face of the leading-end part.

### Effects of the Invention

According to the intraocular lens injector of the present invention, it is possible to form the leading-end side of the plunger to be thin while retaining rigidity, as well as possible to effectively prevent the occurrence of shaft shake during contact with the intraocular lens.

### BRIEF DESCRIPTION OF THE DRAWINGS

FIG. 1 is a perspective view showing the external appearance of an intraocular lens injector of a first embodiment;
FIG. 2 is a plan view showing a state in which an intraocular lens is set in a lens holder;
FIG. 3 is a plan view of a plunger;
FIG. 4 is an external-appearance perspective view showing a plunger in an initial position;
FIG. 5 is an external-appearance perspective view showing a plunger having moved from the initial position to a leading end side;
FIG. 6 is a view showing a shape of an opening of a first embodiment;
FIG. 7 is a view showing the shape of an opening of a second embodiment;
FIG. 8 is a view showing the shape of an opening of a third embodiment;
FIG. 9 is a view showing the shape of an opening of a fourth embodiment; and
FIG. 10 is a view showing the shape of an opening of a fifth embodiment.

### PREFERRED MODE FOR CARRYING OUT THE INVENTION

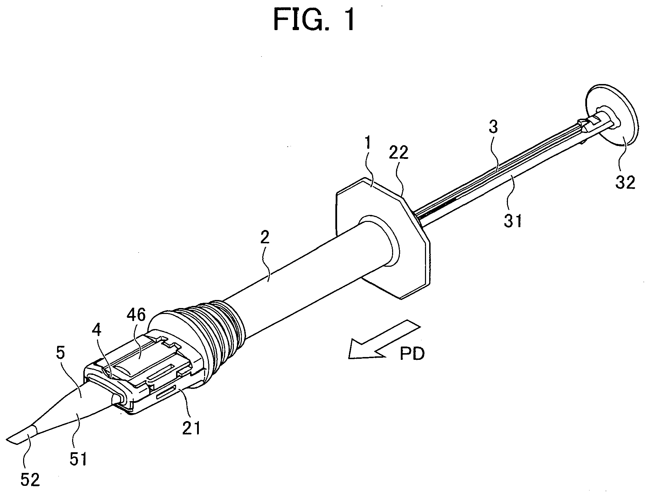

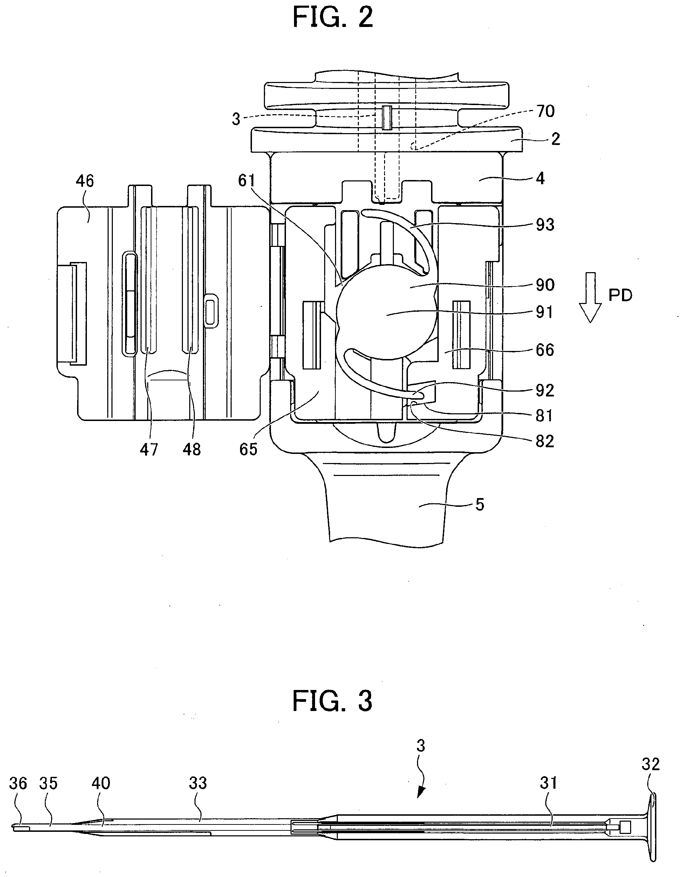

Hereinafter, each preferred embodiment of an intraocular lens injector of the present invention will be explained while referencing the drawings. FIG. 1 is a perspective view showing an external appearance of an intraocular lens injector 1 of a first embodiment. FIG. 2 is a plan view showing a state in which an intraocular lens 90 is set in a lens holder 4. It should be noted that, in the following explanations, the same direction as the axial direction of the plunger 3 is defined as a direction PD, and a side of releasing the intraocular lens in the direction PD is defined as a tip side. In addition, an opposite side to the tip side in the direction PD is defied as a base side. Furthermore, the vertical direction indicates the same direction as a thickness direction of the lens holder 4, and the left/right direction indicates a direction orthogonal to the vertical direction when viewing the lens holder 4 in the direction PD.

The intraocular lens injector 1 shown in FIG. 1 is an insertion tool for an intraocular lens 90 that intraocularly releases the intraocular lens 90 set in the lens holder 4 from a leading end tip 5 by pressing a plunger 3.

Next, a main configuration of the intraocular lens injector 1 will be explained. As shown in FIG. 1, the intraocular lens injector 1 of the present embodiment includes a main body 2, plunger 3, lens holder 4, and leading end tip 5.

The main body 2 is formed in a cylindrical shape having a through-hole for inserting the plunger 3. A plate-shaped fixing part 21 for fixing the lens holder 4 projects at an end face on the leading end side of the main body 2 to the leading end side. On the other hand, a brim part 22 serving as a location at which the user puts a hand is formed at an outer circumferential face on the base end side of the main body 2. As shown in FIG. 2, the through hole is formed at a position at which an opening 70 on the leading end side is in communication with the interior of the lens holder 4.

The plunger 3 is a shaft-like member that pushes the intraocular lens 90 set in the lens holder 4 to the leading end side. The plunger 3 of the present embodiment is formed so that the diameter becomes smaller step-wise as approaching from the base side to the leading end side (refer to FIG. 3). It should be noted that the details of this plunger 3 and the opening 70 formed in the main body 2 will be described later.

The lens holder 4 is for setting the intraocular lens 90 in the intraocular lens injector 1, and is arranged between the main body 2 and the leading end tip 5.

First, the intraocular lens 90 to be set in the lens holder 4 will be explained. As shown in FIG. 2, the intraocular lens 90 includes an optical part 91, first lens support part 92, and second lens support part 93. The optical part 91 is a lens portion functioning as the crystalline lens after intraocular insertion. The first lens support part 92 and second lens support part 93 are formed so as to extend in a curved shape from a lateral face of the optical part 91, and achieve a function of retaining the optical part 91 inside the eye after intraocular insertion. The first lens support part 92 and second lens support part 93 are arranged as to be in a point symmetrical positional relationship with the center of the optical part 91 as the center of symmetry. This optical part 91, first lens support part 92 and second lens support part 93 are formed to be deformable from materials having flexibility.

Next, the configuration of the lens holder 4 will be explained. As shown in FIG. 2, the lens holder 4 of the present embodiment includes a lens holder main body 61, wall parts 65, 66, retaining part 81, and cover 46.

The lens holder main body 61 is formed in a plate shape, and has placement face on which to place the optical part 91 of the intraocular lens 90 at the center of a planar portion.

The wall parts 65, 66 are each formed along the direction PD at both left and right sides of the lens holder main body 61. A space between the interior and exterior of the lens holder main body 61 is partitioned by these wall parts 65, 66, and movement of the intraocular lens in a direction orthogonal to the direction PD is restricted.

The retaining part 81 is at the leading end side of the lens holder main body 61, and is arranged so as to be adjacent to the wall part 66. A recess 82 at which the top and wall part 65 side are opened is formed in the retaining part 81. The intraocular lens 90 is set in the lens holder 4 in a state retaining a leading end side of the first lens support part 92 of the intraocular lens 90 by the recess 82 of the retaining part 81.

The cover 46 is installed in an openable state to the lens holder main body 41, and the interior and exterior of the lens holder 4 are isolated by closing this cover 46. In addition, cover ribs 47, 48 are formed along the direction PB at the inner face of the cover 46. When closing the cover 46, movement in the vertical direction of the intraocular lens 90 is suppressed by the cover ribs 47, 48.

The leading end tip 5 will be explained. The leading end tip 5 includes a nozzle part 51, and a release part 52. The nozzle part 51 has an internal channel communicating with the lens holder 4, and this internal channel is configured so as to narrow as progressing to the release side. The release part 52 is a release opening for releasing the intraocular lens 90 to outside of the intraocular lens injector 1, and is positioned at the leading end of the intraocular lens injector 1. The intraocular lens 90 is released intraocularly from the release part 52 in a state in which the optical part 91 is folded by the inner walls of the internal channel of the nozzle part 51. It should be noted that the insertion operation of the intraocular lens 90 is performed in a state filling an appropriate viscoelastic material or wound treatment drug such as sodium hyaluronate, hydroxypropyl methylcellulose and polyvinyl pyrrolidone into the lens holder 4.

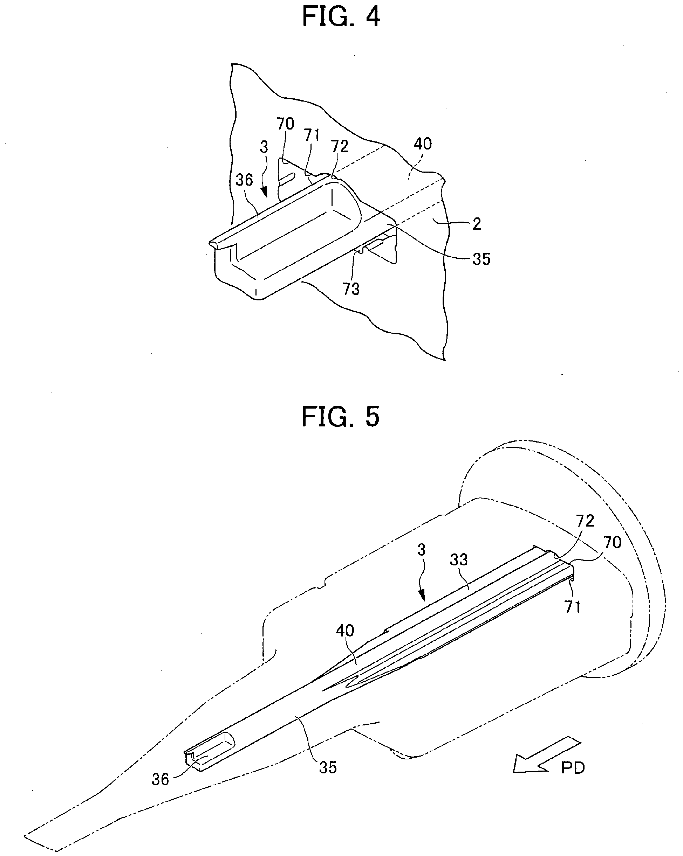

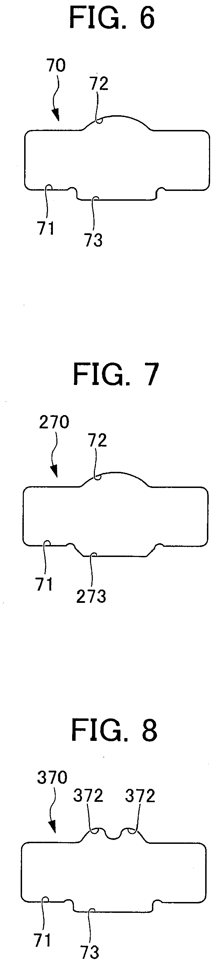

The main body configuration of the intraocular lens injector 1 is as above. Next, a shaft shake prevention structure for the plunger 3 equipped to the intraocular lens injector 1 of the present embodiment will be explained. FIG. 3 is a plan view of the plunger 3. FIG. 4 is an external-appearance perspective view showing the plunger 3 at an initial position of the main body 2. FIG. 5 is an external-appearance perspective view showing the plunger 3 having moved from the initial position to the leading end side. FIG. 6 is a view showing the shape of an opening 70 of the present embodiment.

First, the detailed configuration of the plunger 3 will be explained. As shown in FIG. 3, the plunger 3 includes a base-side shaft part 31, pressing part 32, central-shaft part 33, leading-end-side shaft part 35, L-shaped section 36, and restricted part 40.

The base-end-side shaft part 31 is positioned the most base-end side of the plunger 3. The base-end-side shaft part 31 is a portion that is exposed externally in a state in which the plunger 3 is at the initial position inserted in the main body 2 (refer to FIG. 1). The pressing part 32 is formed in a brim shape at the circumferential face of the base-end side of the base-end-side shaft part 31. The user causes the plunger 3 to move to the leading-end side by pushing this pressing part 32 to the side of the main body 2.

The central-shaft part 33 is connected to the leading-end side of the base-end-side shaft part 31, and is a shaft part of thinner diameter than the base-end-side shaft part 31. As shown in FIG. 5, the central-shaft part 33 is formed in a shape in which the cross-sectional shape thereof is flat. In addition, the thickness direction of the central-shaft part 33 formed to be flat is the same direction as the thickness direction of the lens holder 4 (vertical direction).

The leading-end-side shaft part 35 is connected to the leading end side of the central-shaft part 33, and is formed to be thinner than the central-shaft part 33. The leading-end-side shaft part 35 of the present embodiment has, at the leading end thereof, an L-shaped section 36 in which a cross-sectional shape is L shaped and contacting the second lens support part 93 of the intraocular lens 90 (refer to FIG. 4). When the leading-end-side shaft part 35 contacts with the intraocular lens 90, it becomes possible to push the intraocular lens 90 to the leading end side, while placing a part of the second lens support part 93 in a notched portion of the L-shaped section 36 and folding back to the side of the optical part 91 into an appropriate state.

The restricted part 40 is formed along the axial direction of the plunger 3 from the base-end side of the L-shaped section 36 of the leading-end-side shaft part 35 over substantially the entire region of the central-shaft part 33. The restricted part 40 is formed continuously without being interrupted from the leading-end-side shaft part 35 to the central-shaft part 33, and at the central-shaft part 33 formed to be flat, makes a long and narrow ribbed shape projecting from the flat face thereof in the thickness direction (vertical direction). Then, the cross-sectional shape of the restricted part 40 makes a semi-cylindrical shape. The length in the axial direction of this restricted part 40 is set so that shaft shake is prevented by contacting with the opening 70 in the case of the plunger 3 deviating in the up direction in the state of the initial position described later.

In this way, the plunger 3 of the present embodiment is formed so as to become thinner step-wise as approaching the leading-end side from the base-end side. In addition, the plunger 3 is formed in a curved shape in which the top face on the leading-end side thereof is smooth. In the present embodiment, when viewing in the axial direction, the top face of the L-shaped section 36 is formed in a substantially circular arc shape, and the top face of the restricted part 40 formed from the base-end side of the L-shaped section 36 is configured in a smooth chevron shape from the left-right ends in the left-right direction towards a central part. By the top face of a portion entering the interior of the leading-end tip 5 being formed by a smooth curved face in this way, the leading-end side of the plunger 3 fits with the shape of an inner-cavity top part of the leading-end tip 5, whereby a smooth insertion operation is realized. Furthermore, since the top face on the leading-end side of the plunger 3 is formed by a smooth curved face, a situation in which the inner wall top part of the leading-end tip 5 is damaged by the top face on the leading-end side of the plunger 3 in the course of insertion is also prevented.

Next, the opening 70 will be explained. As shown in FIG. 6, the opening 70 includes a central opening 71, restriction part 72, and bottom-side restriction part 73, which are formed based on the shape of the plunger 3.

The central opening 71 is formed in a substantially rectangular shape that is horizontally long. The central opening 71 of the present embodiment is formed according to the cross-sectional shape of the central-shaft part 33 of the plunger 3, and makes an oblong rectangular shape in the left-right direction in which the corners are rounded.

The restriction part 72 is formed in a notched shape so as to project upwards from a center on the top side (side on one side in the thickness direction) of the central opening 71. The restriction part 72 of the present embodiment is formed in a circular arc shape in which the end face thereof matches the cross-sectional shape of the restricted part 40.

The bottom-side restriction part 73 is formed in a notched shape so as to project downwards from the center of a bottom side (side on the other side in the thickness direction) of the central opening 71. The bottom-side restriction part 73 of the present embodiment is formed in an oblong shape that is horizontally long in the left-right direction in which the corners are rounded to match the shape of the bottom of the L-shaped section 36.

Next, the function of shaft shake prevention by the opening 70 during operation of the plunger 3 will be explained. As shown in FIG. 2, the initial position of the plunger 3 is a state in which a part of the leading-end-side shaft part 35 is exposed at the interior of the lens holder 4, and the L-shaped section 36 of the plunger 3 is positioned more to the base-end side than the second lens support part 93 of the intraocular lens 90. In this initial position, the leading-end face of the plunger 3 is in a state opposing the second lens support part 93.

As mentioned above, the restricted part 40 of the plunger 3 has the length thereof set so as to be restricted by the opening 70 at the initial position; therefore, at the initial position shown in FIG. 4, it is a state in which the restriction part 72 of the opening 70 is opposing with the restricted part 40 of the plunger 3.

In addition, at the initial position, the bottom of the L-shaped section 36 of the plunger 3 is in a state caught in the bottom-side restriction part 73 of the opening 70. In other words, in the state in which the plunger 3 is at the initial position, it enters a state in which shaking orthogonal to the axial direction, which is the vertical direction of the plunger 3, is restricted by the restriction part 72 and the bottom-side restriction part 73 of the opening 70.

In a state in which the plunger 3 is at the initial position, when the plunger 3 is pushed from the initial position to the leading-end side in the direction PD by the user, the L-shaped section 36 of the plunger 3 contacts with the second lens support part 93 positioned at the base-end side of the intraocular lens 90, and enters a state in which the second lens support part 93 is folded to the side of the optical part 91. In the present embodiment, since the restricted part 40 is formed without being interrupted from the leading-end side shaft part 35 to the central shaft part 33, shaking in the axial direction of the plunger 3 will be continually restricted from the initial position until the leading-end face of the plunger 3 contacts the second lens support part 93. Furthermore, since the movement in the left-right direction and down direction of the plunger 3 is restricted by the bottom-side restriction part 73, it becomes possible to appropriately control contact between the plunger 3 and the second lens support part 93 of the intraocular lens 90.

As shown in FIG. 5, when the plunger 3 further advances to the leading-end side, the optical part 91 is folded by the inner wall of the leading-end tip 5, and is inserted into the eye by the release part 52 in this state. The leading-end side shaft part 35 of the plunger 3 is formed to be thinner than the central shaft part 33, and thus it becomes possible to maintain contact thereof for long until the intraocular lens 90 is discharged to outside, without interfering with the inner wall of the leading-end tip 5. Furthermore, by the bottom of the plunger 3 being continually restricted by the bottom-side restriction part 73, stable movement to the axial direction of the plunger 3 is realized.

The following such effects are exerted according to the intraocular lens injector 1 of the first embodiment explained above.

The plunger 3 of the intraocular lens injector 1 of the first embodiment includes: the leading-end side shaft part 35 (leading-end part) having the L-shaped section 36 (contact part) that contacts the intraocular lens 90; the central shaft part 33 (flat part) that is connected to a base-end side of the leading-end side shaft part 35, and is formed flat to be wider than the leading-end side shaft part 35; and the restricted part 40 that is formed from the leading-end side shaft part 35 to the central shaft part 35 along the axial direction of the plunger 3, and projects from the flat face at the central shaft part 33. In addition, the opening 70 in the main body 2 includes a central opening 71 which is opened in accordance with the shape of the central shaft part 33; and a restriction part 72 which is formed so as to project in the thickness direction of the central shaft part 33 from the end face of the central opening 71 in accordance with the shaft of the restricted part 40. Then, the restricted part 40 has a length in the axial direction thereof set so that the L-shaped section 36 is restricted by the restriction part 72 from the initial position prior to starting contact with the second lens support part 93 of the intraocular lens 90 until contacting.

It is thereby possible to form the leading-end side shaft part 35 (leading-end part) to be thin, while raising the rigidity of the plunger 3, by having the central shaft part 33 (flat part) formed to be flat, as well as possible to reliably prevent shaft shake occurring while the plunger 3 contacts with the intraocular lens 90.

In addition, the plunger 3 further includes the bottom (second restricted part) that projects along the axial direction of the plunger 3 at the flat face on an opposite side from the face of the central shaft part 33 at which the restricted part 40 is formed. The opening 70 further includes the bottom-side restriction part 73 (second restriction part) that is arranged on the opposite side to the restriction part 72 sandwiching the central opening 71, and is formed in accordance with the shape of the bottom part of the plunger 3, in which the bottom-side restriction part 73 has a length in the axial direction thereof set so that the L-shaped section 36 is restricted by the bottom-side restriction part 73 from the initial position prior to starting contact with the intraocular lens 90 until contacting.

Since it is thereby possible to restrict movement of the plunger 3 in the thickness direction of the flat part by way of the restriction part 72 and bottom-side restriction part 73 (second restriction part), it is possible to more effectively prevent shaft shake of the plunger 3.

The bottom-side restriction part 73 (second restriction part) is formed continuously from the central shaft part 33 until the leading-end face of the leading-end side shaft part 35 (leading-end face of L-shaped section 36).

From the point of the time in which the plunger 3 is being inserted in the main body 2 to reach the opening 70, since shaft shake of the plunger 3 is prevented by the bottom-side restriction part 73, it is thereby possible to more finely set the initial position of the plunger 3.

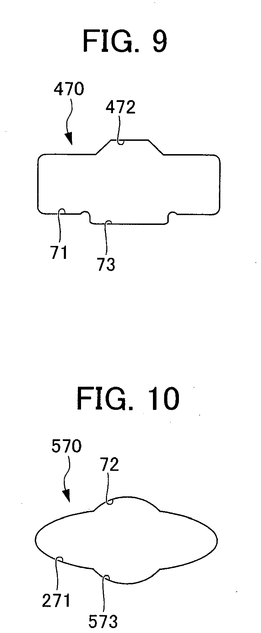

Although the configuration of the intraocular lens injector of the first embodiment has been explained above, the shapes of the plunger 3 and opening 70 are not to be limited to the configurations of the above-mentioned embodiment, and modifications are possible as appropriate. Next, another embodiment modifying the shape of the opening will be explained. FIG. 7 is a view showing the shape of an opening 270 of a second embodiment. FIG. 8 is a view showing the shape of an opening 370 of a third embodiment. FIG. 9 is a view showing the shape of an opening 470 of a fourth embodiment. FIG. 10 is a view showing the shape of an opening 570 of a fifth embodiment. It should be noted that the same reference symbols are assigned for configurations that are similar to the first embodiment, and explanations thereof may be omitted.

As shown in FIG. 7, the opening 270 of the second embodiment differs from the first embodiment in the shape of a bottom-side restriction part 273. In the second embodiment, the bottom-side restriction part 273 is formed in a chamfered rectangular shape (substantially trapezoidal shape). In the second embodiment, the plunger is also configured in a shape in which the bottom of the cross-sectional shape of the L-shaped section is chambered so as to correspond to the bottom-side restriction part 273.

As shown in FIG. 8, the opening 370 of the third embodiment differs from the first embodiment in the shape of a restriction part 372. In the third embodiment, two of the restriction parts 372 are formed so as to project from the top side of the central opening 71. In the third embodiment, two of the restricted parts of the plunger are formed in a shape forming along the axial direction.

As shown in FIG. 9, the opening 470 of the fourth embodiment differs from the first embodiment in the shape of a restriction part 472. In the fourth embodiment, the shape of the restriction part 472 is formed in a substantially trapezoidal shape. In the fourth embodiment, the cross-sectional shape of the restricted part of the plunger is configured in a substantially trapezoidal shape.

As shown in FIG. 10, the opening 570 of the fifth embodiment differs from the first embodiment in the shape of a central opening 271 and bottom-side restriction part 573. In the fifth embodiment, the shape of the central opening 271 is formed in an oblong elliptical shape. In addition, the end face of the bottom-side restriction part 573 is formed in a circular arc shape. In the fifth embodiment, the plunger is configured in a shape with the bottom part of the L-shaped section formed in a circular arc shape, and the shape of the central shaft part in accordance with the shape of the central opening 271.

Although each of the preferred embodiments of an intraocular lens injector of the present invention have been explained above, the present invention is not to be limited to the aforementioned embodiments, and further modifications are possible as appropriate. For example, in the above-mentioned embodiments, although the shapes of the opening and plunger are formed in substantially the same shapes, the shapes of the opening and plunger may differ so long as being able to prevent shaft shake of the plunger by way of the opening. For example, it is also possible to use a plunger having a circumferential face formed in a circular arc shape as in the restricted part 40 of the first embodiment, also in the opening 470 of the fourth embodiment shown in FIG. 9. In this case, the shape of the opening 470 and the shape of the plunger 3 will be adjusted so that the restricted part 40 is restricted by the restriction part 473.

In the above-mentioned embodiments, although the leading-end face of the plunger 3 is formed in an L shape, the shape of the plunger can be modified as appropriate. Furthermore, the leading-end face of the plunger 3 can also be formed in a circular shape, rectangular shape or elliptical shape.

Although the intraocular lens injector 1 in which the lens holder 4 and main body 2 are configured separately has been explained as an example in the above-mentioned embodiments, it is not necessarily limited to this configuration, and it is possible to make a configuration in which the lens holder is formed integrally with the main body. In addition, the configuration of the lens holder 4 is not necessarily limited to the configuration of the present embodiment. So long as having the configuration of the present invention that prevents shaft shake of the plunger, the intraocular lens injection can modify the respective configurations such as the main body, leading-end tip, lens holder and plunger thereof.

### EXPLANATION OF REFERENCE NUMERALS

1 intraocular lens injector
2 main body
3 plunger
4 lens holder
33 central shaft part (flat part, second restricted part)
35 leading-end side shaft part (leading-end part)
36 L-shaped section (contact part, second restricted part)
40 restricted part
70 opening
71 central opening
72 restriction part
73 bottom-side restriction part (second restriction part)
90 intraocular lens

## Claims

1. An intraocular lens injector comprising:
a main body formed in a cylindrical shape;
a lens holder that is disposed at a leading-end side of the main body, and in which an intraocular lens is set; and
a plunger that is inserted in the main body, and pushes out the intraocular lens set in the lens holder from an opening formed in the leading-end side of the main body to a leading-end side,
wherein the plunger includes:
a leading-end part having a contact part that contacts with the intraocular lens;
a flat part that is connected to a base-end side of the leading-end part, and is formed to be flat and wider than the leading-end part; and
a restricted part that is formed along an axial direction of the plunger from the leading-end part to the flat part, and projects from a flat face at the flat part,
wherein the opening includes:
a central opening that is opened in accordance with the shape of the flat part; and
a restriction part that is formed so as to project in a thickness direction of the flat part from an end face of the central opening in accordance with the shape of the restricted part, and
wherein the restricted part has a length in the axial direction thereof set so as to be restricted by at least the restriction part from an initial position prior to the contact part starting contact with the intraocular lens until contacting.

2. The intraocular lens injector according to claim 1,
wherein the plunger further includes a second restriction part that projects along the axial direction of the plunger at a flat face on a side opposite to a face of the flat part on which the restricted part is formed,
wherein the opening further includes a second restriction part that is disposed on a side opposite to the restriction part to sandwich the central opening, and is formed in accordance with a shape of the second restricted part of the plunger, and
wherein the second restricted part has a length in the axial direction thereof set so as to be restricted by at least the second restriction part from an initial position prior to the contact part starting contact with the intraocular lens until contacting.

3. The intraocular lens injector according to claim 2, wherein the second restricted part is formed continuously from the flat part until an end face of the leading-end part.
